Europäisches Patentamt

European Patent Office

Office européen des brevets

(11) Veröffentlichungsnummer: **0 414 116 B1**

(12) **EUROPÄISCHE PATENTSCHRIFT**

(45) Veröffentlichungstag der Patentschrift: **22.06.94**

(21) Anmeldenummer: **90115637.2**

(22) Anmeldetag: **16.08.90**

(51) Int. Cl.⁵: **C07C 255/50**, C07C 253/34

(54) **Verfahren zur Trennung von ortho-, meta- und para-Tolunitril aus ternären Isomerengemischen.**

(30) Priorität: **24.08.89 DE 3927916**

(43) Veröffentlichungstag der Anmeldung:
**27.02.91 Patentblatt 91/09**

(45) Bekanntmachung des Hinweises auf die
Patenterteilung:
**22.06.94 Patentblatt 94/25**

(84) Benannte Vertragsstaaten:
**BE DE ES FR GB**

(56) Entgegenhaltungen:

CHEMICAL ABSTRACTS, vol. 82, no. 21, 26.
Mai 1975, Columbus, Ohio, US; abstract no.
139792W, Seite 594, Spalte 2; & JP-A-49 116
032

THE JOURNAL OF ORGANIC CHEMISTRY, Bd.
51, Nr. 13, 27. Juni 1986; F.J.WEIGERT: "Isomerization of methylbenzonitriles catalyzed
by HZSM-5"

(73) Patentinhaber: **BASF Aktiengesellschaft
Carl-Bosch-Strasse 38
D-67063 Ludwigshafen(DE)**

(72) Erfinder: **Steck, Werner, Dr.
Auerstrasse 4
D-6700 Ludwigshafen(DE)**
Erfinder: **Rust, Harald
Dudostrasse 57
D-6730 Neustadt(DE)**
Erfinder: **Lermer, Helmut, Dr.
Bockenheimer Strasse 12
D-6700 Ludwigshafen(DE)**
Erfinder: **Fritz, Naeumann, Dr.
Rathenaustrasse 3a
D-6800 Mannheim 1(DE)**

**Beschreibung**

Die Vorliegende Erfindung betrifft ein Verfahren zur Trennung von ortho-, meta- und para-Tolunitril aus ternären Isomerengemischen durch Kombination aus Destillation und Kristallisation durch Ausfrieren einer Komponente.

Aus F. R. Weigert, J. Org. Chem. 1986, 51, 2653 - 2655 ist die Isomerisierung von Methylbenzonitrilen (Tolunitrilen) in der Gasphase an einem Aluminiumzeolithen vom Typ ZSM-5 in der aciden H-Form bekannt. Bei 500°C und Kontaktzeiten von etwa 3 Sekunden soll dabei das thermodynamische Gleichgewicht mit 46 % ortho-, 34 % meta- und 20 % para-Tolunitril eingestellt werden, ohne daß Nebenprodukte in größeren Mengen entstehen.

Andere Zeolithe wie die weitporigen Zeolithe HY, H-Mordenit und H-Omega sollen die Isomerisierung nicht katalysieren.

Bei allen Verfahren, die zu ternären Isomerengemischen, nämlich zu ortho-, meta- und para-Tolunitril führen, ist eine wirtschaftliche Nutzung bisher unterblieben. Die Ursache dafür liegt in den geringen Siedepunktsdifferenzen der drei isomeren Tolunitrile, die eine destillative Trennung im technischen Maßstab bisher als wirtschaftlich nicht durchführbar erscheinen lassen.

Es bestand daher die Aufgabe auf wirtschaftliche Weise aus ternären oder binären Tolunitrilgemischen die reinen Tolunitrile zu gewinnen und ein Verfahren zur Herstellung geeigneter ternärer Ausgangsisomerengemische zu entwickeln.

Demgemäß wurde ein Verfahren zur Trennung von ortho-, meta- und para-Tolunitril aus ternären Isomerengemischen gefunden, welches dadurch gekennzeichnet ist, daß man aus diesen Gemischen bei Drücken von $10^2$ bis $10^5$ Pa und einem Rücklaufverhältnis von 1:1 bis 200:1 das ortho-Tolunitril destillativ abtrennt und das verbleibende binäre Gemisch aus meta- und para-Tolunitril destillativ auf über 75 mol-% para-Tolunitril aufkonzentriert und das para-Tolunitril bei Temperaturen kleiner als 26°C ausfriert. Ferner wurde ein Verfahren zur Herstellung von geeigneten ternären Tolunitril-Isomerengemischen durch Isomerisierung von reinem ortho-, meta- oder para-Tolunitril oder deren Gemische bei 380 bis 580°C an Zeolithkatalysatoren gefunden.

Die Trennung des ternären Gemisches durch Destillation (hier Rektifikation) kann diskontinuierlich und kontinuierlich erfolgen. Es hat sich gezeigt, daß sich Betriebsdrücke von $10^2$ bis $10^5$ Pa, vorzugsweise $10^3$ bis $3 \cdot 10^4$ Pa, insbesondere um $10^4$ Pa, zur Trennung gut eignen. Es können Trennkolonnen gemäß dem Stand der Technik wie Füllkörperkolonnen, auch in der Ausführung als Kolonnen mit Längsteilung, oder Bodenkolonnen eingesetzt werden. Sehr gute Ergebnisse lassen sich beispielsweise durch kontinuierliche Aufarbeitung des ternären Rohgemisches in zwei Kolonnen oder in einer Kolonne mit Längsteilung bei entsprechender Bodenzahl erzielen.

Außerdem hat es sich gezeigt, daß zur Reingewinnung von para-Tolunitril aus diesen Isomerengemischen eine Kombination von destillativer und kristallisativer Trennung auf besonders einfachem und wirtschaftlichem Wege möglich ist. Dazu wird zunächst das o-Tolunitril bei Drucken von $10^2$ bis $10^5$ Pa, vorzugsweise $10^2$ bis $3 \cdot 10^4$ Pa abdestilliert. Aus dem verbleibenden binären Gemisch von meta- und para-Tolunitril wird dann das m-Tolunitril gleichfalls bei Drücken von $10^2$ bis $10^5$ Pa, vorzugsweise $10^2$ bis $3 \cdot 10^4$ Pa soweit abdestilliert, daß das verbleibende Gemisch aus meta- und para-Tolunitril aus über 75, z.B. zwischen 75 und 95 mol-%, vorzugsweise aus über 80 % an para-Tolunitril besteht. Aus solchen para-reichen Gemischen kann reines para-Tolunitril zwischen -25 und +25°C, bevorzugt bei -10 bis +20°C auf überraschend einfache Weise durch Kristallisation gewonnen werden. Bei Bedarf kann die Kristallisation auch durch den Zusatz von Impfkristallen ausgelöst werden kann. In der verbleibenden, überstehenden Mutterlauge reichert sich meta-Tolunitril an. Je nach gewünschter Reinheit des para-Tolunitrils kann die Kristallisation durch Schmelzen des jeweiligen Kristallisats und nachfolgende erneute Kristallisation beliebig oft wiederholt werden.

Das für die erfindungsgemäße Trennung erforderliche Gemisch der drei Tolunitrilisomeren kann durch Isomerisierung von reinen Tolunitrilen oder beliebigen Tolunitrilgemischen, die aus zwei oder allen Isomeren bestehen, hergestellt werden. Solche binäre oder ternäre Isomerengemische fallen beispielsweise auch bei der erfindungsgemäßen destillativen oder kristallisativen Trennung an und können in eine Isomerisierungsreaktion zurückgeführt werden.

Zur Herstellung von Isomerengemischen als besonders geeignet hat sich die Isomerisierung von ortho-, meta- und para-Tolunitril oder deren Gemische in der Gasphase bei 380 bis 580°C an Zeolithkatalysatoren, insbesondere an Aluminosilikatzeolithen oder Galliumsilikatzeolithen der Pentasilfamilie, erwiesen, da sich hiermit bereits in einem Durchgang ternäre Isomerengemische herstellen lassen, deren Zusammensetzung im Bereich des thermodynamischen Gleichgewichts von 46 mol-% ortho, 34 mol-% meta und 20 mol-% para liegen kann.

Die für die erfindungsgemäße Isomerisierung geeigneten Reaktionsbedingungen liegen bei 380 bis 580°C. Die Reaktion läßt sich beispielsweise in der Gasphase bei Normaldruck in einem Festbett, Fließ- oder Wirbelbett ausführen. Bei einer Gesamtgewichtsbelastung WHSV von 0,1 bis 2,5 h$^{-1}$ (Dimension der WHSV = g Einsatzgemisch an Tolunitrilen je g Katalysator und Stunde) lassen sich gute Ergebnisse erzielen. Es hat sich dabei als zweckmäßig erwiesen die Reaktion in Gegenwart von Inertgasen wie Stickstoff oder Edelgasen durchzuführen. Erfolgt die Isomerisierung in Gegenwart von Wasserstoff, so können auf Kosten der Selektivität auch höhere Umsätze erhalten werden.

Ferner ist es möglich die Isomerisierung unter Druck auszuführen. In Abhängigkeit von der Temperatur und dem Druck können sich die Toluntril-Isomeren dabei in der Gasphase, Flüssigphase oder in überkritischem Zustand befinden. Für die Umsetzung kann dabei Druck von $10^5$ bis $10^7$ Pa, bevorzugt $2 \cdot 10^6$ bis $7 \cdot 10^6$ Pa eingestellt werden.

Die Isomerisierung erfolgt mit hoher Selektivität. Als Nebenprodukte können sich geringe Mengen an Toluol, Benzonitril und Dimethylbenzonitrilen bilden.

Die für das erfindungsgemäße Verfahren geeigneten Zeolithe vom Pentasiltyp weisen als sekundäre Struktureinheit (= Secondary Building Unit, Grundbaustein der Zeolith-Struktur) einen aus $TO_4$-Tetraedern aufgebauten Fünfring auf, wobei als T-Kationen, Si, Al oder Ga verwendet werden können. Die Definition der Pentasil-Zeolithe stammt von G. T. Kokotailo und W. M. Meier, Chem. Soc. Spec. Publ. 1980, 33, Seite 133 - 139. Pentasilzeolithe sind durch ein hohes molares $SiO_2/Al_2O_3$-Verhältnis - auch Modul genannt - gekennzeichnet, sowie durch Porengrößen, die zwischen denen der Zeolithe vom Typ A und denen vom Typ X oder Y liegen. Für das erfindungsgemäße Verfahren haben sich beispielsweise Pentasil-Zeolithe mit $SiO_2/Al_2O_3$-Verhältnissen zwischen 25 und 10.000 als gut geeignet erwiesen. Die Herstellung dieser Pentasil-Zeolithe wird u.a. in den US-PS 3 702 886 (ZSM-5), US-PS 3 709 979 (ZSM-11), US-A 4 061 724 (Silicalite®) und US-A 4 073 865, beschrieben. Auch gehören hierzu die isotaktischen Pentasil-Zeolithe nach EP-A-34 727.

Der Aluminosilikatzeolith wird z.B. aus einer Aluminiumverbindung, vorzugsweise $Al(OH)_3$ oder $Al_2(SO_4)_3$ und einer Siliciumkomponente, wie hochdisperses Siliciumdioxid, Kieselgel oder Kieselsol in wäßriger Aminlösung, insbesondere in Polyaminen wie 1,6-Diaminohexan oder 1,3-Diaminopropan oder Triethylentetramin-Lösung mit oder insbesondere ohne Alkali- oder Erdalkalizusatz bei 100 bis 220°C unter autogenem Druck hergestellt. Die erhaltenen Aluminosilikatzeolithe haben je nach Wahl der Einsatzstoffmengen ein $SiO_2/Al_2O_3$-Verhältnis von 15 bis 10.000.

Auch lassen sich derartige Aluminosilikatzeolithe in etherischem Medium wie Diethylenglykoldimethylether, in alkoholischem Medium wie Methanol oder 1,4-Butandiol oder in Wasser synthetisieren.

Auch Galliumsilikatzeolithe oder Zeolithe, welche als dreiwertiges Gitterion gleichzeitig Gallium und Aluminium enthalten, sind geeignet.

Als zur raschen Gleichgewichtseinstellung bei der Isomerisierung der Tolunitrile gut geeignet haben sich feinteilige Zeolithe erwiesen deren Kristallgrößen etwa 3 $\mu$m nicht überschreiten und bevorzugt kleiner als etwa 1,5 $\mu$m sind. Die Kristallgröße läßt sich gemäß dem Stand der Technik leicht elektronenmikroskopisch entweder in Transmission (TEM) oder mit REM (Rasterelektronenmikroskopie) bestimmen.

Die pulverförmig anfallenden Zeolithe können nach ihrer Synthese, Isolierung, Trocknung bei 100 bis 160°C und Calcinierung bei 450 bis 550°C, mit einem Bindemittel im Massenverhältnis Zeolith zu Bindemittel 90:10 bis 40:60 Gew.-% zu Formkörpern wie Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:10, $SiO_2$, $TiO_2$, Gemische aus hochdispersem $SiO_2$ und hochdispersem $Al_2O_3$ sowie Ton. Nach der Verformung werden die Extrudate oder Preßlinge bei 110°C getrocknet und bei Temperaturen um 500°C calciniert.

Man erhält auch gut geeignete Katalysatoren, wenn der isolierte Zeolith direkt nach der Trocknung verformt wird und erst nach der Verformung einer Calcinierung unterworfen wird. Die Pentasilzeolithe können in reiner Form, ohne Binder, als Stränge oder Tabletten eingesetzt werden, wobei als Verstrangungs- oder Peptisierungsmittel z.B. Ethylcellulose, Stearinsäure, Kartoffelstärke, Ameisensäure, Oxalsäure, Essigsäure, Salpetersäure, Ammoniak, Amine und Graphit oder deren Gemische verwendet werden können.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen, und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C zu regenerieren. Die Zeolithe erhalten dadurch in der Regel ihre Anfangsaktivität zurück.

Um eine möglichst hohe Selektivität, hohen Umsatz sowie lange Standzeiten zu erreichen, ist es gegebenenfalls vorteilhaft, die Zeolithe zu modifizieren, wobei der Gehalt an den Modifizierungselementen so zu bemessen ist, daß eine ausreichende Aktivität der Katalysatoren gewährleistet ist.

Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden beispielsweise Seltenerdmetalle, wie Lanthan und/oder Cer, oder Edelmetalle, wie Pd, eingesetzt.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium-, Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat, einem Carbonat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine weitere Möglichkeit der Modifizierung besteht darin, daß man das zeolithische Material - vor oder nach seiner Verformung - einer Behandlung mit Säuren wie Salzsäure, Flußsäure und Phosphorsäure und/oder Wasserdampf unterwirft. Dabei geht man z.B. so vor, daß man Zeolithe in Pulverform mit 1 n Phosphorsäure 1 h bei 80°C behandelt. Nach der Behandlung wird mit Wasser gewaschen, bei 110°C/16 h getrocknet und beispielsweise bei 500°C für 10 Stunden calciniert.

Die nachstehend beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelgut eingesetzt werden.

Die isomerenreinen Tolunitrile werden benötigt als Ausgangs- und Zwischenprodukte für eine Vielzahl organischer Synthesen (Römpps Chemie-Lexikon; 8. Auflage, 1988, S. 4290) und gemäß US-A-3 231 600, Spalte 1, 2. Absatz als Lösungsmittel für verschiedene Zwecke.

Herstellung der Katalysatoren

Katalysatoren 1 und 2

Es wurden handelsübliche ZSM-5 Katalysatoren in der H-Form mit folgenden Eigenschaften eingesetzt:

| Katalysator | $SiO_2/Al_2O_3$ | Kristallgröße [$\mu$m] |
|---|---|---|
| 1 | 57 | 0,75 |
| 2 | 57 | <0,05 |

Katalysator 3

Ein Galliumsilikatzeolith des Pentasil-Typs wird in einer hydrothermalen Synthese aus 1730 g hochdispersem $SiO_2$, 469 g Natriumgallat-Lösung (12,7 Gew.-% $Ga_2O_3$, 12,2 Gew.-% Na), 6000 g wäßriger 1,6-Diaminohexan-Lösung (50 Gew.-% Amin) und 21.000 g Wasser bei 150°C unter autogenem Druck in einem Rührautoklaven hergestellt. Nach Abfiltrieren und Auswaschen wird das kristalline Reaktionsprodukt bei 100°C 24 h getrocknet und bei 500°C 24 h calciniert. Dieser Galliumsilikatzeolith setzt sich zusammen aus 91,9 Gew.-% $SiO_2$ und 3,06 Gew.-% $Ga_2O_3$. Die Kristallgröße liegt bei <0,1 um.

Mit diesem Material werden durch Verformen mit pyrogener Kieselsäure (8 Massenteile : 2 Massenteile) und einem Verformungshilfsmittel 2-mm-Stränge hergestellt, die bei 110°C 16 h getrocknet und bei 500°C 16 h calciniert werden.

Die Stränge werden viermal mit 20 gew.-%iger $NH_4$Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,0045 Gew.-%.

Katalysator 4

Ein Aluminosilikatzeolith vom ZSM-5-Typ wird unter hydrothermalen Bedingungen bei autogenem Druck und 160°C aus 1500 g pyrogener Kieselsäure, 84 g Aluminiumsulfat-18-hydrat, 240 g Natriumhydroxid, 980 g Tetra-n-propylammoniumbromid und 18000 g Wasser in einem Rührautoklaven hergestellt. Nach Abfiltrieren und Auswaschen wird das kristalline Reaktionsprodukt bei 110°C/24 h getrocknet und bei 500°C 24 h calciniert. Dieser Zeolith ist durch folgende Zusammensetzung charakterisiert: 85,4 Gew.-% $SiO_2$, 1,00 Gew.-% $Al_2O_3$, 0,75 Gew.-% Na. Die Kristallgröße beträgt 2 $\mu$m.

Mit diesem Zeolith werden durch Verformen mit einem Verformungshilfsmittel 2-mm-Stränge hergestellt, die bei 110°C/16 h getrocknet und bei 500°C 16 h calciniert werden.

Die Stränge werden viermal mit 20 gew.-%iger $NH_4$Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,015 Gew.-%.

### Katalysator 5

Ein Aluminosilikatzeolith vom ZSM-5-Typ wird unter hydrothermalen Bedingungen bei autogenem Druck und 175°C aus 1000 g pyrogener Kieselsäure, 48 g Natriumaluminat (58,6 Gew.-% $Al_2O_3$, 40,0 Gew.-% $Na_2O$), 44 g Natriumhydroxid, 1690 g wäßriger Tetra-n-propylammoniumhydroxid-Lösung (20%ig) und 16600 g Wasser in einem Rührautoklaven hergestellt. Nach Abfiltrieren und Auswaschen wird das kristalline Reaktionsprodukt bei 110°C/24 h getrocknet und bei 500°C 24 h calciniert. Dieser Zeolith ist durch folgende Zusammensetzung charakterisiert: 85,8 Gew.-% $SiO_2$, 2,3 Gew.-% $Al_2O_3$, 0,40 Gew.-% Na. Die Kristallgröße beträgt 3 $\mu$m.

Mit diesem Material werden durch Verformen mit pyrogener Kieselsäure (8 Massenteile : 2 Massenteile) und einem Verformungshilfsmittel 2-mm-Stränge hergestellt, die bei 110°C 16 h getrocknet und bei 500°C 16 h calciniert werden.

Die Stränge werden viermal mit 20 gew.-%iger $NH_4$Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,002 Gew.-%.

### Katalysator 6

Ein Aluminosilikatzeolith vom ZSM-11-Typ wird unter hydrothermalen Bedingungen bei autogenem Druck und 160°C aus 10900 g Wasser, 6300 g Natrium-Wasserglas (8,0 Gew.-% $Na_2O$, 26,5 Gew.-% $SiO_2$), 180 g Aluminiumsulfat-18-hydrat, 600 g Schwefelsäure konz. und 600 g 1,8-Diaminooctan in einem Rührautoklaven hergestellt. Nach Abfiltrieren und Auswaschen wird das kristalline Reaktionsprodukt bei 110°C 24 h getrocknet und bei 500°C 24 h calciniert. Dieser Zeolith ist durch folgende Zusammensetzung charakterisiert: 81,8 Gew.-% $SiO_2$, 1,82 Gew.-% $Al_2O_3$. Die Kristallgröße beträgt 0,2 $\mu$m.

Mit diesem Material werden durch Verformen mit Pseudoboehmit (6 Massenteile : 4 Massenteile) 2-mm-Stränge hergestellt, die bei 110°C 16 h getrocknet und bei 500°C 16 h calciniert werden.

Die Stränge werden viermal mit 20 gew.-%iger $NH_4$Cl-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,0026 Gew.-%.

### Katalysator 7

Ein Aluminosilikatzeolith vom Pentasil-Typ wird unter hydrothermalen Bedingungen bei autogenem Druck und 150°C aus 650 g hochdispersem $SiO_2$, 203 g $Al_2(SO_4)_3$ x 18 $H_2O$ in 11300 g einer wäßrigen 1,6-Diaminohexan-Lösung (42 Gew.-% Amin) in einem Rührautoklaven hergestellt. Nach Abfiltrieren und Auswaschen wird das kristalline Reaktionsprodukt bei 110°C/24 h getrocknet und bei 500°C/24 h calciniert. Dieser Aluminosilikatzeolith enthält 92,6 Gew.-% $SiO_2$ und 4,6 Gew.-% $Al_2O_3$. Die Kristallgröße beträgt 0,25 $\mu$m.

Mit diesem Material werden durch Verformen mit einem Verformungshilfsmittel 2-mm-Stränge hergestellt, die bei 110°C 16 h getrocknet und bei 500°C 16 h calciniert werden.

Die Stränge werden mit einer wäßrigen $Ce(NO_3)_3$ x 6 $H_2O$-Lösung getränkt, bei 130°C 2 h getrocknet und bei 540°C 2 h calciniert. Der Ce-Gehalt beträgt 1,6 Gew.-%.

### Katalysator 8

Die undotierten Stränge von Katalysator 7 werden mit einer wäßrigen $La(NO_3)_3$ x 6 $H_2O$-Lösung getränkt, bei 130°C 2 h getrocknet und bei 540°C 2 h calciniert. Der La-Gehalt beträgt 1,8 Gew.-%.

Katalysator 9 A

Ein Aluminosilikatzeolith vom Pentasil-Typ wird unter hydrothermalen Bedingungen bei autogenem Druck und 184°C aus 700 g hochdispersem $SiO_2$ und 196 g $Al_2(SO_4)_3$ x 18 $H_2O$ in 12,2 kg einer wäßrigen Triethylentetramin-Lösung (38 Gew.-% Amin) in einem Rührautoklaven hergestellt. Nach Abfiltrieren und Auswaschen wird das kristalline Reaktionsprodukt bei 110°C/24 h getrocknet und bei 500°C/24 h calciniert. Dieser Aluminosilikatzeolith enthält 92,9 Gew.-% $SiO_2$ und 3,8 Gew.-% $Al_2O_3$. Die Kristallgröße beträgt 8,5 $\mu$m.

Mit diesem Material werden durch Verformen mit einem Verformungshilfsmittel 2 mm-Stränge hergestellt, die bei 110°C 16 h getrocknet und bei 500°C 16 h calciniert werden.

Katalysator 9 B

Die undotierten Stränge von Katalysator 9 A werden mit einer wäßrigen $Pd(NO_3)_2$-Lösung getränkt, bei 130°C 1 h getrocknet und bei 540°C 1 h calciniert. Der Pd-Gehalt beträgt 2,05 Gew.-%.

Katalysator 10

Ein Molekularsieb vom Silicalith-Typ wird unter hydrothermalen Bedingungen bei autogenem Druck und 160°C aus 2250 g pyrogener Kieselsäure, 324 g Natriumhydroxid, 1500 g Tetra-n-propylammoniumbromid und 27000 g Wasser in einem Rührautoklaven hergestellt. Nach Abfiltrieren und Auswaschen wird das kristalline Reaktionsprodukt bei 110°C/24 h getrocknet und bei 500°C 24 h calciniert. Dieser Molekularsieb ist durch folgende Zusammensetzung charakterisiert: 95,5 Gew.-% $SiO_2$, 0,0020 Gew.-% $Al_2O_3$. Die Kristallgröße beträgt 6 $\mu$m.

Mit diesem Molekularsieb werden durch Verformen mit einem Verformungshilfsmittel 2-mm-Stränge hergestellt, die bei 110°C/16 h getrocknet und bei 500°C 16 h calciniert werden. Die Stränge werden viermal mit 20 gew.-%iger $NH_4Cl$-Lösung (15 ml Lösung/g Formkörper) bei 80°C in einem Steigrohr ausgetauscht. Anschließend wird chloridfrei gewaschen. Die Stränge werden bei 110°C 10 h getrocknet und anschließend bei 500°C 5 h calciniert. Der Na-Gehalt beträgt nun 0,09 Gew.-%.

Katalysatoren für Vergleichsbeispiele

Katalysator 11

Katalysator 11 ist ein handelsübliches $Al_2O_3$ (D 10-10®)

Katalysator 12

Katalysator 12 ist ein handelübliches $SiO_2$ (D 11-10®)

Katalysator 13

Handelsüblicher Mordenit ZEOLON 900 H von Norton.

Katalysator 14

Handelsüblicher US-Y-Zeolith in der H-Form

Katalysator 15

Ein SAPO-11 wird synthetisiert, indem man 200 g Orthophosphorsäure (98 Gew.-%) mit 417 g Aluminiumtriisopropylat und 60 g Kieselsol (30 Gew.-% $SiO_2$) in 927 g Wasser homogen mischt; zu dieser Mischung werden 91,5 g Di-n-propylamin hinzugefügt. Danach wird bei 200°C 96 h unter autogenem Druck in einem Rühr-Autoklaven umgesetzt. Das abfiltrierte und gewaschene Siliciumaluminiumphosphat wird getrocknet bei 110°C und calciniert bei 500°C. Die Zusammensetzung des SAPO-11 ist 40,4 Gew.-% $Al_2O_3$, 49,5 Gew.-% $P_2O_5$ und 1,87 Gew.-% $SiO_2$. Die Kristallgröße ist kleiner als 0,5 $\mu$m.

Mit diesem Material werden durch Verformen mit einem Verformungshilfsmittel 2-mm-Stränge hergestellt, die bei 110°C/16 h getrocknet und bei 500°C/16 h calciniert werden.

Technische und physikalische Daten der Tolunitrile.

Tabelle I

| Siedepunkte der Tolunitrile bei Normaldruck in [°C] | | |
|---|---|---|
| ortho | 205 | CRC Handbook of Chemistry and Physics 68th Edition |
| meta- | 213 | CRC Handbook of Chemistry and Physics 68th Edition |
| para- | 218 | CRC Handbook of Chemistry and Physics 68th Edition |

Tabelle II

| Schmelzpunkt der Tolunitrile (MG 117.2) | | |
|---|---|---|
| | Smp. [°C] | Quelle |
| ortho | -13,5 | CRC Handbook of Chemistry and Physics 68th Edition |
| meta- | -23 | CRC Handbook of Chemistry and Physics 68th Edition |
| para- | 29,5 | CRC Handbook of Chemistry and Physics 68th Edition |

I. Isomerisierung von Tolunitrilen

Beispiele 1 bis 18

Gasphasenreaktionen zur Isomerisierung von Tolunitrilen

Die Reaktionen in der Gasphase wurden bei Normaldruck unter isothermen Bedingungen in einem Rohrreaktor (Wendel, 0,6 cm Innendurchmesser, Rohrlängen zwischen 90 cm und 180 cm) im einmaligen Durchgang oder mehrfachen Durchgang mittels einer Kreisgasfahrweise an einem Katalysatorfestbett bei 400 bis 550°C durchgeführt. Die Reaktionsprodukte wurden durch übliche Techniken (GC/MS, NMR oder Siedepunk) charakterisiert.

Zur Herstellung von geeigneten Isomerengemischen im vorstehend beschriebenen Rohrreaktor werden die Einsatzprodukte, z.B. o-Tolunitril, bei Normaldruck mit einer Dosierpumpe unter gleichzeitigem Inertisieren mit einem Stickstoffstrom dem Reaktor kontinuierlich zugeführt und darin vor Erreichen des Katalysatorbettes verdampft und dann gasförmig über den Katalysator geleitet.

Das mit Katalysator beschickte Festbett wird dabei je nach Fahrweise entweder aufsteigend oder absteigend von den gasförmig vorliegenden Edukten und Reaktionsprodukten durchströmt. Bei der Fahrweise mit einmaligen Durchgang (Reaktionstyp 1) sind beispielsweise Gesamtbelastungen WHSV von etwa 0,2 bis 2 $h^{-1}$ geeignet. Bei der Kreislauffahrweise (Reaktionstyp 2) wurden beispielsweise 15 bis 25 g Katalysator im Festbett verwendet. In einem Glaskolben wurden 30 bis 50 ml Tolunitril vorgelegt und mittels einer Dosierpumpe dann das Tolunitril mit einem Volumenstrom von 50 ml/h im Kreislauf umgepumpt und jeweils vor dem Passieren des Katalysatorfestbettes verdampft.

Nach beendeter Isomerisierung wird der Reaktionsaustrag kondensiert und nach der in Stunden (h) angegebenen Laufzeit gaschromatographisch analysiert. Die Umsätze (U) und Selektivitäten (S) sind in den Tabellen in Flächenprozenten angegeben. Die Ausbeuten werden mit dem Symbol A und Tolunitril mit TN abgekürzt.

R-Typ = Reaktionstyp 1 = einmaliger Durchsatz; Reaktionstyp 2 = Kreisgasfahrweise; T = Temperatur; V = Vergleichsbeispiel.

Als Nebenprodukte wurden im wesentlichen nur Benzonitril, Dimethylbenzonitrile und Toluol nachgewiesen.

A) Isomerisierung von o-Tolunitril

B) Isomerisierung von m-Tolunitril

C) Isomerisierung eines binären Gemisches von 50 % o-Tolunitril und 50 % m-Tolunitril

D) Isomerisierung von ternären Isomerengemischen, welche bei der destillativen Auftrennung (Beispiele 20 bis 22) angefallen sind.

Die Ergebnisse sind in den Tabellen A bis D zusammengefaßt.

Tabelle A

Isomerisierung von o-Tolunitril

| Bei-spiel | Katalysator Nr. | Einwaage [g] | R-Typ | Laufzeit in h | T [°C] | Umsatz o-TN | m-TN | p-TN | m+p |
|---|---|---|---|---|---|---|---|---|---|
| 1 | 2 | 4 | 1 | 2 | 500 | 15,3 | 71,4 | 11,2 | 82,6 |
| 2 | 2 | 20 | 1 | 2 | 500 | 55,0 | 53,9 | 27,2 | 81,6 |
| | | | | 12 | 500 | 33,1 | 75,4 | 21,1 | 96,5 |
| | 2 x regeneriert bei 500°C | | | 2 | 500 | 55,6 | 54,3 | 28,6 | 82,9 |
| | | | | 6 | 500 | 44,0 | 67,2 | 27,5 | 94,7 |
| V1 | 14 | 4 | 1 | 2 | 500 | 1,7 | 44,5 | 2,9 | 47,4 |
| 3 | 2 | 20 | 2 | 6 | 500 | 49,2 | 60,0 | 27,8 | 87,8 |
| | | | | 22 | | 55,2 | 56,8 | 30,3 | 87,1 |
| 4 | 2 | 20 | 2 | 5 | 400 | 19,0 | 65,3 | 24,3 | 85,6 |
| | | | | 21 | | 29,4 | 68,0 | 22,4 | 90,4 |
| 5 | 1 | 20 | 2 | 2 | 500 | 46,8 | 60,0 | 29,9 | 89,9 |
| | | | | 18 | | 57,2 | 54,9 | 30,9 | 85,8 |
| 6 | 3 | 20 | 2 | 17 | 500 | 54,0 | 39,5 | 14,5 | 54,0 |
| 7 | 4 | 20 | 2 | 23 | 500 | 52,9 | 58,6 | 31,4 | 90,0 |
| 8 | 5 | 20 | 2 | 22 | 500 | 49,1 | 57,6 | 28,5 | 86,1 |
| 9 | 6 | 20 | 2 | ·23 | 500 | 48,3 | 46,2 | 30,2 | 94,6 |
| 10 | 7 | 20 | 2 | 21 | 500 | 29,5 | 74,8 | 16,9 | 91,7 |
| 11 | 8 | 20 | 2 | 22 | 500 | 28,8 | 73,8 | 16,3 | 90,5 |
| 12 | 9A | 20 | 2 | 17 | 500 | 31,4 | 59,5 | 30,9 | 90,4 |
| 13 | 9B | 20 | 2 | 22 | 500 | 19,9 | 59,4 | 30,8 | 91,2 |
| 14 | 10 | 20 | 2 | 23 | 500 | 14,6 | 58,0 | 30,2 | 88,2 |
| V2 | 11 | 20 | 2 | 23 | 500 | 6,9 | 5,8 | 0,7 | 6,5 |
| V3 | 12 | 20 | 2 | 71 | 500 | 2,9 | 46,9 | 10,8 | 57,8 |
| V4 | 13 | 20 | 2 | 21 | 500 | 10,3 | 54,2 | 11,1 | 55,3 |
| V5 | 14 | 20 | 2 | 21 | 500 | 2,5 | 26,9 | 0,0 | 26,9 |
| V6 | 15 | 20 | 2 | 23 | 500 | 6,8 | 55,5 | 18,2 | 78,5 |

Tabelle B

| Isomerisierung von m-Tolunitril | | | | | | | Selektivitäten | | |
|---|---|---|---|---|---|---|---|---|---|
| Beispiel | Katalysator | | R-Typ | Laufzeit in h | T [°C] | Umsatz m-TN | | | |
| | Nr. | Einwaage [g] | | | | | o-TN | p-TN | o+p |
| 14 | 9 | 20 | 2 | 17 | 510 | 50,0 | 45,7 | 45,1 | 90,8 |

8

Tabelle C

Isomerisierung eines binären Gemisches von 50 % o-Tolunitril und 50 % m-Tolunitril

Alle Analysenangaben in Flächenprozenten

| Bei-spiel | Katalysator Nr. | Einwaage [g] | R-Typ | Laufzeit in h | T [°C] | o-TN | m-TN | p-TN | m+p +o |
|---|---|---|---|---|---|---|---|---|---|
| 15 | 9 | 22 | 2 | Einsatz-produkt | – | 50,0 | 50,0 | 00,0 | 100,0 |
|  |  |  |  | 17 | 490 | 49,1 | 33,9 | 13,6 | 99,0 |

Tabelle D

Isomerisierung von ternären Isomerengemischen, welche bei der destillativen Auftrennung (Beispiele 20 bis 23) angefallen sind.

Alle Analysenangaben sind Flächenprozente

| Bei-spiel | Katalyssator Nr. | Einwaage [g] | R-Typ | Laufzeit in h | T [°C] | o-TN | m-TN | p-TN | m+p +o |
|---|---|---|---|---|---|---|---|---|---|
| 16 | 2 | 20 | 2 | Einsatz-produkt | – | 89,8 | 10,0 | 0,2 | 100,0 |
|  |  |  |  | 1 | 500 | 58,9 | 30,4 | 8,4 | 97,7 |
|  |  |  |  | 4 |  | 39,9 | 39,2 | 17,0 | 96,1 |
|  |  |  |  | 20 |  | 33,2 | 43,3 | 18,8 | 95,3 |
| 17 | 2 | 20 | 2 | Einsatz-produkt | – | 80,6 | 0,2 | 19,2 | 100,0 |
|  |  |  |  | 6 | 500 | 33,1 | 40,0 | 19,6 | 92,7 |
|  |  |  |  | 22 |  | 31,9 | 42,3 | 17,9 | 92,1 |

Beispiel 19

Isomerisierung in flüssiger Phase unter Druck

In einem Rohrreaktor mit 9 mm Durchmesser und 25 cm Länge wurden 5 g des Katalysators Nr. 2 (handelsüblicher ZSM-5) in Form von Katalysatorsplit einer Korngröße von 0,1 bis 0,5 mm eingebaut. Der Katalysator wurde zunächst eine Stunde in einem Stickstoffstrom bei 450°C getempert. Dann wurde der Reaktorausgang auf ein Überströmventil ($5 \cdot 10^6$ Pa) geschaltet, der Reaktor über eine Pumpe mit ortho-Tolunitril befüllt und dann zwei Stunden ein Produktstrom von 15 g/h o-Tolunitril über den bei 450°C befindlichen Katalysator geleitet, der Produktaustrag aufgefangen und gaschromatographisch analysiert.
Die Ergebnisse sind in Tabelle E zusammengefaßt.

Tabelle E

| Beispiel | Katalyssator | | Laufzeit in h | T [°C] | Umsatz o-TN | Selektivitäten | | |
|---|---|---|---|---|---|---|---|---|
| | Nr. | Einwaage [g] | | | | m-TN | p-TN | m + p |
| 19 | 2 | 5 | 2 | 450 | 21,8 | 64,8 | 15,2 | 80 |

II. Destillative Gewinnung der Tolunitrile aus Isomerengemischen

Beispiele 20 bis 22: Rektifikation von Tolunitril-Isomeren

Beispiel 20

980 g Rohgemisch mit ca. 0,2 % Benzonitril, 75 % o-Tolunitril, 14,8 % m-Tolunitril und 10 % p-Tolunitril wurden in einer Füllkörperkolonne (Glas, 140 cm lang, 25 mm Durchmesser, gefüllt mit Edelstahl-Drahtnetzwendeln von 3 mm Durchmesser, theoretische Bodenzahl beträgt ca. 20 bis 30) bei $10^4$ Pa bzw. $10^3$ Pa und einem Rückflußverhältnis von 30 : 1 in Fraktionen rektifiziert. Durch Erniedrigung des Betriebsdruckes gegen Ende der Destillation von $10^4$ auf $10^3$ Pa sollten temperaturabhängige Reaktionen verhindert werden.

Die Ergebnisse sind in Tabelle F zusammengefaßt.

Konzentrationsangaben sind Flächenprozente aus GC.

Ergebnis: Die im Ausgangsgemisch enthaltenen Komponenten destillieren gemäß ihren Siedepunkten nacheinander mindestens 99 %ig über Kopf der Kolonne ab. p-Tolunitril befindet sich dem Rückstand mit mehr als 98 % Reinheit und kann ggf. durch Kristallisation weiter gereinigt werden. Die Zwischenlaufanteile werden zwangsläufig um so geringer, je größer die Trennleistung der Kolonne ist.

Tabelle F

| Frak-tion Nr. | Druck Pa | Temperatur | | Gewicht | | Massenanteile | | Benzo-nitril | o- | m- | p- |
|---|---|---|---|---|---|---|---|---|---|---|---|
| | | Sumpf | Kopf | g | Sg | % | S% | | | Tolunitril | |
| Ausgangsprobe | | | | | | | | 0,2 | 75,0 | 14,8 | 10,0 |
| 1 | 104 | 143 | 128 | 24,0 | 24,0 | 2,45 | 2,45 | 9,1 | 89,9 | 0,8 | -- |
| 2 | 104 | 142 | 129 | 63,0 | 87,0 | 6,43 | 8,88 | 0,9 | 98,6 | 0,5 | -- |
| 3 | 104 | 141 | 129 | 88,0 | 175,0 | 8,98 | 17,86 | 0,03 | 99,6 | 0,3 | -- |
| 4 | 104 | 141 | 128 | 207,0 | 382,0 | 21,12 | 38,98 | - | 99,9 | 0,1 | -- |
| 5 | 104 | 143 | 126 | 239,0 | 621,0 | 24,39 | 63,37 | - | 99,9 | 0,02 | -- |
| 6 | 104 | 142 | 127 | 91,0 | 712,0 | 9,29 | 72,65 | - | 100,0 | -- | -- |
| 7 | 104 | 142 | 129 | 23,0 | 735,0 | 2,35 | 75,0 | - | 95,6 | 4,3 | 0,3 |
| 8 | 104 | 144 | 133 | 51,0 | 786,0 | 5,20 | 80,20 | - | 10,0 | 89,8 | 0,1 |
| 9 | 104 | 210 | 132 | 50,0 | 836,0 | 5,10 | 85,31 | - | 0,07 | 99,2 | 0,7 |
| 10 | 103 | 146 | 81 | 42,0 | 878,0 | 4,29 | 89,59 | - | 0,2 | 80,6 | 19,2 |
| 11 | 103 | 200 | 82 | 29,0 | 907,0 | 2,96 | 92,55 | - | - | 40,2 | 59,8 |
| Rückstand | | | | 73,0 | 980,0 | 7,45 | 100,0 | - | - | 1,2 | 98,6 |

Beispiel 21

996 g Rohgemisch mit ca. 0,3 % Benzonitril, 45,7 % o-Tolunitril, 33,7 % m-Tolunitril und 20,2 % p-Tolunitril wurden in der gleichen Kolonne wie im Beispiel 20 fraktioniert rektifiziert. Es zeigt sich, daß die für die Trennung verantwortlichen Hauptparameter wie Mindestrücklaufverhältnis, Querschnittsbelastung und Trennwirkung nicht im Grenzbereich sein dürfen, da sonst, wie hier ersichtlich, keine spezifikationsgerechte Trennung möglich ist. Die Ergebnisse sind in Tabelle G zusamengefaßt Konzentrationsangaben sind Flächenprozente aus GC.

Tabelle G

| Frak-tion Nr. | Druck Pa | Temperatur Sumpf (°C) | Kopf (°C) | Gewicht g | Sg | Massenanteile % | S% | Benzo-nitril | o- Tolunitril | m- Tolunitril | p- Tolunitril |
|---|---|---|---|---|---|---|---|---|---|---|---|
| Ausgangsgemisch | | | | | | | | 0,3 | 45,7 | 33,7 | 20,3 |
| 1 | $10^4$ | 143 | 130 | 14,0 | 14,0 | 1,41 | 1,41 | 14,6 | 79,3 | 1,7 | 0,6 |
| 2 | $10^4$ | 143 | 125 | 219,0 | 233,0 | 21,99 | 23,39 | 0,6 | 98,9 | 0,3 | 0,06 |
| 3 | $10^4$ | 142 | 125 | 197,0 | 430,0 | 19,78 | 43,17 | – | 99,7 | 0,2 | 0,06 |
| 4 | $10^4$ | 142 | 131 | 38,0 | 486,0 | 3,82 | 46,99 | – | 52,6 | 47,3 | – |
| 5 | $10^4$ | 143 | 132 | 30,0 | 498,0 | 3,01 | 50,00 | – | 11,1 | 88,8 | 0,02 |
| 6 | $10^4$ | 143 | 132 | 87,0 | 585,0 | 8,73 | 58,73 | – | 2,1 | 97,7 | 0,2 |
| 7 | $10^4$ | 145 | 132 | 47,0 | 632,0 | 4,72 | 63,45 | – | 0,3 | 99,4 | 0,3 |
| 8 | $10^3$ | 113 | 62 | 87,0 | 719,0 | 8,73 | 72,19 | – | – | 88,8 | 11,2 |
| 9 | $10^3$ | 113 | 62 | 36,0 | 755,0 | 3,61 | 75,80 | – | – | 76,2 | 23,6 |
| 10 | $10^3$ | 113 | 64 | 99,0 | 854,0 | 9,94 | 85,74 | – | – | 43,9 | 56,0 |
| 11 | $10^3$ | 113 | 64 | 45,0 | 899,0 | 4,52 | 90,26 | – | – | 10,5 | 89,5 |
| 12 | $10^3$ | 129 | 64 | 65,0 | 964,0 | 6,53 | 96,79 | – | – | 5,2 | 94,7 |
| Rückstand | | 200 | | 32,0 | 996,0 | 3,21 | 100,0 | – | – | 1,8 | 97,9 |

Beispiel 22

1938 g Rohgemisch mit der gleichen Zusammensetzung wie im Beispiel 20 wurden in der gleichen Kolonne wie in Beispiel 1 fraktioniert rektifiziert. Die analytischen Ergebnisse der Fraktionen zeigen die optimale Trennung der isomeren Tolunitrile.

Die Ergebnisse sind in Tabelle H zusamengefaßt. Konzentrationsangaben sind Flächenprozente aus GC.

Tabelle H

| Frak-tion Nr. | Druck Pa | Temperatur Sumpf (°C) | Temperatur Kopf (°C) | Gewicht g | Gewicht Sg | Massenanteile % | Massenanteile S% | Benzo-nitril | o- Tolunitril | m- Tolunitril | p- Tolunitril |
|---|---|---|---|---|---|---|---|---|---|---|---|
| Ausgangsgemisch | | | | | | | | 0,3 | 45,7 | 33,7 | 20,3 |
| 1 | $10^4$ | 138 | 126 | 49,0 | 49,0 | 2,53 | 2,53 | 5,9 | 93,2 | - | - |
| 2 | $10^4$ | 141 | 116 | 74,0 | 123,0 | 3,82 | 6,35 | 2,7 | 96,9 | - | - |
| 3 | $10^4$ | 140 | 126 | 77,0 | 200,0 | 3,97 | 10,32 | 1,3 | 98,3 | - | - |
| 4 | $10^4$ | 141 | 126 | 86,0 | 286,0 | 4,44 | 14,76 | 0,3 | 99,3 | - | - |
| 5 | $10^4$ | 140 | 126 | 77,0 | 363,0 | 3,97 | 18,73 | - | 99,7 | - | - |
| 6 | $10^4$ | 142 | 126 | 82,0 | 445,0 | 4,23 | 22,96 | - | 100,0 | - | - |
| 7 | $10^4$ | 142 | 128 | 83,0 | 528,0 | 4,28 | 27,24 | - | 100,0 | - | - |
| 8 | $10^4$ | 142 | 128 | 92,0 | 620,0 | 4,75 | 31,99 | - | 100,0 | - | - |
| 9 | $10^4$ | 142 | 128 | 96,0 | 716,0 | 4,95 | 36,95 | - | 100,0 | - | - |
| 10 | $10^4$ | 142 | 130 | 97,0 | 813,0 | 5,01 | 41,95 | - | 98,2 | 1,8 | - |
| 11 | $10^4$ | 142 | 133 | 89,0 | 902,0 | 4,59 | 46,54 | - | 42,4 | 57,6 | - |
| 12 | $10^4$ | 144 | 134 | 79,0 | 981,0 | 4,08 | 50,62 | - | 7,8 | 91,9 | 0,2 |
| 13 | $10^4$ | 144 | 134 | 82,0 | 1063,0 | 4,23 | 54,85 | - | 1,4 | 98,2 | 0,4 |
| 14 | $10^4$ | 145 | 134 | 93,0 | 1156,0 | 4,80 | 59,65 | - | 0,2 | 99,2 | 0,5 |
| 15 | $10^4$ | 146 | 134 | 104,0 | 1260,0 | 5,37 | 65,02 | - | - | 99,3 | 0,7 |
| 16 | $10^4$ | 146 | 133 | 91,0 | 1351,0 | 4,70 | 69,71 | - | - | 98,3 | 1,7 |
| 17 | $10^4$ | 145 | 135 | 97,0 | 1448,0 | 5,01 | 74,72 | - | - | 93,0 | 6,9 |
| 18 | $10^4$ | 145 | 136 | 90,0 | 1538,0 | 4,64 | 79,36 | - | - | 41,6 | 58,4 |
| 19 | $10^4$ | 144 | 136 | 20,0 | 1558,0 | 1,03 | 80,39 | - | - | 9,8 | 90,1 |
| 20 | $10^4$ | 154 | 136 | 126,0 | 1684,0 | 6,50 | 86,89 | - | - | 1,6 | 98,4 |
| 21 | $10^4$ | 215 | 136 | 58,0 | 1742,0 | 2,99 | 89,89 | - | - | - | 100,0 |
| Rückstand | | | | 105,0 | 1847,0 | 5,42 | 95,30 | - | - | - | 100,0 |
| Hold up | | | | 91,0 | 1938,0 | 4,70 | 100,0 | - | - | - | >99,5 |

III. Gewinnung von Tolunitrilen durch Kristallisation

Beispiel 24

Bei der Destillation wurde ein Einsatzprodukt aus 16,6 % m-Tolunitril und 83,4 % p-Tolunitril erhalten. Das binäre Isomerengemisch wurde 12 h bei 15°C gehalten und danach die entstandenen Kristalle abgetrennt. Die Kristalle bestanden zu 97,9 % aus p-Tolunitril und zu 2,1 % aus m-Toluntril. Ein bei 5°C durchgeführter Versuch führte zu Kristallen mit 97,3 % p-Tolunitril und 2,7 % m-Tolunitril.

Beispiel 25

Zwei aus der Destillation erhaltenen binäre Gemische von m- und p-Tolunitril wurden bei 22°C auskristallisiert und sowohl die erhaltenen Kristalle als auch die Mutterlauge mit GC analysiert.
Die Ergebnisse sind in Tabelle K zusammengefaßt.

Tabelle K

| Zusammensetzung | m-Tolunitril [%] | p-Tolunitril [%] |
|---|---|---|
| Einsatzgemisch 1 | 10,5 | 89,5 |
| Kristalle | 0,9 | 99,1 |
| Mutterlauge | 15,1 | 84,9 |
| Einsatzgemisch 2 | 5,2 | 94,8 |
| Kristalle | 0,3 | 99,7 |
| Mutterlauge | 15,1 | 93,9 |

Beispiel 26

Ein flüssiges Gemisch aus 90 % p-Tolunitril und 10 % m-Tolunitril wurde durch Abkühlen auf 0°C vollständig auskristallisiert und danach die erhaltenen Kristalle wieder langsam innerhalb 12 Stunden auf 20°C erwärmt. Dabei schmilzt ein Teil der Kristalle unter Bildung einer Mutterlauge. Aus dem entstandenen Gemisch der verbliebenen Kristalle und der Mutterlauge wurden die Kristalle abgetrennt und gaschromatographisch analysiert.
Die Ergebnisse sind in Tabelle L zusammengefaßt.

Tabelle L

| | m-Tolunitril | p-Tolunitril |
|---|---|---|
| Kristalle | 0,9 % | 99,1 % |
| Mutterlauge | 14,9 % | 85,1 % |

**Patentansprüche**

1. Verfahren zur Trennung von ortho-, meta- und para-Tolunitril aus ternären Isomerengemischen, dadurch gekennzeichnet, daß man aus diesen Gemischen bei Drücken von $10^2$ bis $10^5$ Pa und einem Rücklaufverhältnis von 1:1 bis 200:1 das ortho-Tolunitril destillativ abtrennt und das verbleibende binäre Gemisch aus meta- und para-Tolunitril destillativ auf über 75 mol-% para-Tolunitril aufkonzentriert und das para-Tolunitril bei Temperaturen von unterhalb 26°C ausfriert.

2. Verfahren nach Anspruch 1, dadurch gekennzeichnet, daß man die Destillation bei Drücken von $10^3$ bis $3 \cdot 10^4$ Pa durchführt.

3. Verfahren nach Anspruch 1, dadurch gekennzeichnet, daß man bei der Destillation ein Rücklaufverhältnis von 5:1 bis 50:1 einstellt.

**4.** Verfahren nach Anspruch 1, dadurch gekennzeichnet, daß man die ternären Isomerengemische durch Isomerisierung von reinem ortho-, meta- oder para-Tolunitril oder deren Gemische bei 380 bis 580°C an Zeolithkatalysatoren erhält.

**5.** Verfahren nach Anspruch 4, dadurch gekennzeichnet, daß die Isomerisierung in der Gasphase bei 380 bis 580°C erfolgt.

**6.** Verfahren nach Anspruch 4, dadurch gekennzeichnet, daß die Isomerisierung in flüssiger Phase bei Drücken von $10^5$ bis $10^7$ Pa durchgeführt wird.

**7.** Verfahren nach Anspruch 4, dadurch gekennzeichnet, daß man als Zeolithkatalysatoren Alumino- oder Galliumzeolithe des Pentasiltyps verwendet.

**8.** Verfahren nach Anspruch 4, dadurch gekennzeichnet, daß man als Zeolitkatalysatoren Alumino- oder Galliumzeolithe des Pentasiltyps verwendet, deren Kristallgröße 0,05 bis 3 $\mu$m beträgt.

**9.** Verfahren nach Anspruch 4, dadurch gekennzeichnet, daß man als Zeolitkatalysatoren Alumino- oder Galliumzeolithe des Pentasiltyps verwendet, welche mit Edelmetallen oder mit Metallen der Seltenen Erden dotiert sind.

## Claims

**1.** A process for separating ortho-, meta- and para-tolunitrile from ternary mixtures of isomers, which comprises removing ortho-tolunitrile from these mixtures by distillation under pressures of from $10^2$ to $10^5$ Pa and with a reflux ratio of from 1:1 to 200:1, and distilling the remaining binary mixture of meta- and para-tolunitrile to concentrate to more than 75 mol-% para-tolunitrile, and freezing out the para-tolunitrile at below 26°C.

**2.** A process as claimed in claim 1, wherein the distillation is carried out under pressures of from $10^3$ to 3 x $10^4$ Pa.

**3.** A process as claimed in claim 1, wherein a reflux ratio of from 5 : 1 to 50 : 1 is employed in the distillation.

**4.** A process as claimed in claim 1, wherein the ternary mixtures of isomers are obtained by isomerization of pure ortho-, meta- or para-tolunitrile or mixtures thereof at from 380 to 580°C on zeolite catalysts.

**5.** A process as claimed in claim 4, wherein the isomerization is carried out in the gas phase at from 380 to 580°C.

**6.** A process as claimed in claim 4, wherein the isomerization is carried out in the liquid phase under pressures of from $10^5$ to $10^7$ Pa.

**7.** A process as claimed in claim 4, wherein alumino or gallium zeolites of the pentasil type are used as zeolite catalysts.

**8.** A process as claimed in claim 4, wherein alumino or gallium zeolites of the pentasil type with a crystal size of from 0.05 to 3 $\mu$m are used as zeolite catalysts.

**9.** A process as claimed in claim 4, wherein alumino or gallium zeolites of the pentasil type which have been doped with noble metals or with rare earth metals are used as zeolite catalysts.

## Revendications

**1.** Procédé de séparation de l'ortho-, du méta- et du para-toluonitrile à partir de mélanges d'isomères ternaires, caractérisé en ce que l'on sépare l'ortho-toluonitrile par distillation de ces mélanges à des pressions de $10^2$ à $10^5$ Pa et sous un rapport de reflux de 1:1 à 200:1 et on concentre le mélange binaire résiduel, constitué de méta- et para-toluonitrile, par distillation jusqu'à plus de 75% molaires de para-toluonitrile et on congèle le para-toluonitrile à des températures inférieures à 26 °C.

2. Procédé suivant la revendication 1, caractérisé en ce que l'on entreprend la distillation à des pressions de $10^3$ à $3.10^4$ Pa.

3. Procédé suivant la revendication 1, caractérisé en ce que l'on règle le rapport de reflux de 5:1 à 50:1 au cours de la distillation.

4. Procédé suivant la revendication 1, caractérisé en ce que l'on obtient les mélanges d'isomères ternaires par l'isomérisation d'ortho-, de métha- ou de para-toluonitrile pur, ou de leurs mélanges à 380-580 °C sur des catalyseurs à zéolites.

5. Procédé suivant la revendication 4, caractérisé en ce que l'isomérisation s'effectue à 380-580 °C en phase gazeuse.

6. Procédé suivant la revendication 4, caractérisé en ce que l'on entreprend l'isomérisation en phase liquide et à des pressions de $10^5$ à $10^7$ Pa.

7. Procédé suivant la revendication 4, caractérisé en ce que l'on utilise des aluminozéolites ou des galliozéolites du type pentasile à titre de catalyseurs zéolitiques.

8. Procédé suivant la revendication 4, caractérisé en ce que l'on utilise, à titre de catalyseurs zéolitiques, des aluminozéolites ou des galliozéolites du type pentasile, dont la taille des cristaux varie de 0,05 à 3 $\mu$m.

9. Procédé suivant la revendication 4, caractérisé en ce que l'on utilise, à titre de catalyseurs zéolitiques, des aluminozéolites ou des galliozéolites du type pentasile, qui ont été dopés avec des métaux nobles ou avec des métaux des terres rares.